(19) Europäisches Patentamt
European Patent Office
Office européen des brevets

(11) Publication number: **0 051 321**
**B1**

(12) # EUROPEAN PATENT SPECIFICATION

(45) Date of publication of patent specification: **26.03.86**

(21) Application number: **81110264.9**

(22) Date of filing: **31.10.79**

(60) Publication number of the earlier application in accordance with Art. 76 EPC: **0 012 208**

(51) Int. Cl.$^4$: **C 07 D 241/04,** C 07 D 209/86 // C07D403/06, A61K31/495

(54) **Cis-dimethylpiperazines for the preparation of N-substituted carbazoles.**

(30) Priority: **01.11.78 GB 42845/78**
**02.03.79 US 16914**

(43) Date of publication of application:
**12.05.82 Bulletin 82/19**

(45) Publication of the grant of the patent:
**26.03.86 Bulletin 86/13**

(84) Designated Contracting States:
**GB**

(56) References cited:
**DE-A-2 339 396**
**FR-A-1 167 510**

**CHEMICAL ABSTRACTS, vol. 68, June 17, 1968, page 11027, abstract 114435e, COLUMBUS, OHIO (US)**

**ARZNEIMITTELFORSCHUNG, vol. 12, no. 9, 1962, AULENBURG (DE) E.L. ANDERSON et al.: "Synthesis of Phenothiazines", pages 937-942**

(73) Proprietor: **THE WELLCOME FOUNDATION LIMITED**
**183-193 Euston Road**
**London NW1 2BP (GB)**

(72) Inventor: **Harfenist, Morton**
**Route 6 Box 405**
**Chapel Hill North Carolina 27514 (US)**
Inventor: **Joyner, Charles Thomas**
**Route 8 Box 183H**
**Raleigh North Carolina (US)**

(74) Representative: **Sandmair, Kurt, Dr. et al**
**Patentanwälte Schwabe, Sandmair, Marx**
**Stuntzstrasse 16**
**D-8000 München 80 (DE)**

**EP 0 051 321 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

The present invention relates to compounds usable as intermediates in the preparation of N-substituted carbazoles being the subject matter of the parent patent 0012208 having valuable anti-aggression and antipsychotic properties.

Prior Art

Australian Patent No. 201630 and French Patent No. 1167510 describe as having anti-epileptic properties carbazoles of formula (I):

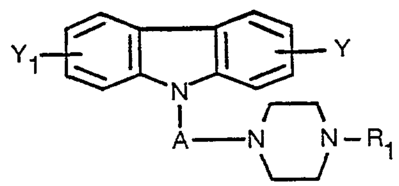

(I)

wherein A represents a divalent saturated aliphatic hydrocarbon radical with a straight or branched chain containing 2 to 6 carbon atoms, $R_1$ represents a hydrogen atom or a lower alkyl or an aryl or araliphatic group, Y and $Y_1$ are respectively a hydrogen or halogen atom or a lower alkyl, alkoxy, aryl or aryloxy group, the ring containing Y or $Y_1$ may contain substituents additional to Y and $Y_1$ and one or more of the carbon atoms of the piperazine ring may carry a substituent in the form of a methyl group.

The FR—A—1,167,510 describes carbazoles of the general formula IV

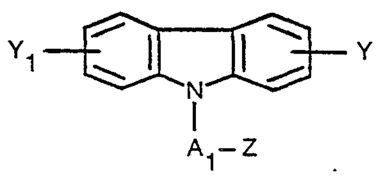

(IV)

wherein $Y_1$ and Y are i.a. hydrogen, $A_1$ is an alkylene radical containing 2—6 carbon atoms and Z is a reactive ester radical, i.a. a halogen atom or a sulphonyloxy residue, for example p-toluenesulphonyloxy. The piperazines used for the preparation of the carbazoles differ from the cis-dimethylpiperazines of the present invention.

The DE—A—2,339,396 i.a. concerns carbazoles of formula III

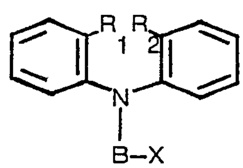

(III)

wherein $R_1$ and $R_2$ together constitute a bond, B is an alkylene group having 2—5 carbon atoms and X is a reactive group, for example halogen, a mesyloxy- or tosyloxy group. The preparation of these carbazoles does not start from the cis-dimethylpiperazines of the present invention.

Description of the Invention

It has now been found that cis-dimethylpiperazines of formula (II)

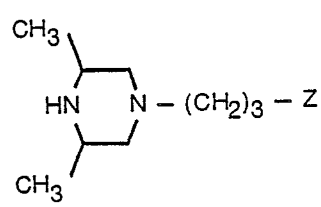

(II)

wherein Z is selected from halo, arylsulfonyloxy and alkylsulfonyloxy, or a salt thereof can be used for the preparation of 9-[3-(3,5-cis-dimethylpiperazino)propyl]carbazole (sometimes named as cis-9-[3-(3,5-dimethylpiperazinyl)propyl]carbazole or 9-[3-(cis-3,5-dimethyl-1-piperazinyl)-propyl]carbazole) of formula (A)

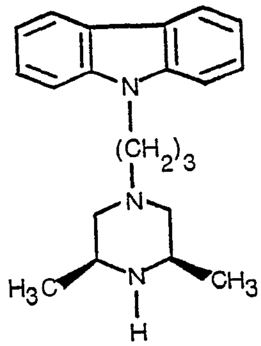

(A)

hereinafter referred to as the "Carbazole", it's pharmaceutically acceptable salts and pharmaceutically acceptable solvates of it's pharmaceutically acceptable salts. These compounds exhibit unexpectedly valuable anti-aggressive and anti-psychotic properties rendering them useful for the treatment of aggression and psychoses in humans. The compounds obtainable from the compounds of the present application unexpectedly do not exhibit the toxic side-effects, e.g. dyslexia or sedative action associated with anti-psychotics such as chlorpromazine and the like.

The "Carbazole" is particularly valuable since its anti-psychotic and anti-aggressive action is substantially free from undesirable side-effects such as sedation, catalepsy and extrapyramidal dysfunction associated with currently used anti-psychotics such as the phenothiazines. An indication that the "Carbazole" does not interfere with extrapyramidal function is its failure to antagonise apomorphine induced behaviour in the rat (see Andien, N.E., *J. Psychiat. Res.*, *11*, 97, 1974. There is also significantly less anti-cholinergic and anti-histamine activity associated with the "Carbazole's" action than with the phenothiazines.

While the useful biological properties of the "Carbazole" reside in the base moiety, the latter is capable of forming salts (i.e. acid addition salts), and all such salts are included within the scope of the parent invention. For therapeutic purposes the acid moiety of such salts is preferably pharmaceutically acceptable to the intended recipient. As examples of such pharmaceutically acceptable salts may be mentioned (a) those formed with inorganic acids, for example the sulphate and hydrogen halides such as the hydrochloride, and (b) those formed with organic acids such as the acetate, citrate, fumarate, lactate (for example the *DL*-lactate), malate (for example the *L*-malate), maleate, succinate and tartrate (for example the L-tartrate) as well as alkanesulphonates such as the methanesulphonate and arylsulphonates such as the *p*-toluenesulphonate. The "Carbazole" base may be converted to salts thereof, and the salts converted to the base or to salts of other acids, by means well known in the art. Thus, salts which are not pharmaceutically acceptable are of value in the preparation of salts which are. In addition the salts of the "Carbazole" base can form well-defined solvates such as hydrates and solvates of alcohols (for example methanol and ethanol) and all such solvates are also included within the scope of the parent invention.

The "Carbazole" may be synthesised by any method known in the art for the synthesis of compounds having an analogous structure.

1. A preferred synthesis involves linking the alkylene chain to either or both of the carbazole and piperazine rings. This may be done by reacting a compound of formula (II)

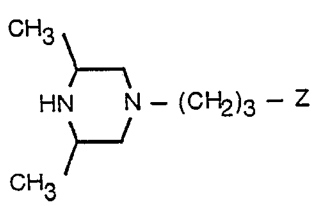

(II)

wherein Z is selected from halo (preferably chloro), arylsulphonyloxy (preferably *p*-toluenesulphonyloxy) or alkylsulphonyloxy (preferably methanesulphonyloxy) with carbazole.

Although compounds (II) will react directly with carbazole it is advantageous to use a carbazole derivative; particularly suitable derivatives are those with alkali or alkaline earth metals, especially lithium, sodium, potassium, zinc, cadmium and calcium, although carbazole magnesium halide, e.g. iodide, may also be used.

The alkali metal carbazoles are conveniently prepared by reacting carbazole with the appropriate alkali metal hydride or amide in an inert, preferably polar, aprotic solvent such as liquid ammonia, dimethylformamide or dimethylsulphoxide; other solvents which may be suitable include hydrocarbons, e.g., alkanes, cycloalkanes and aromatic compounds. The same reaction medium may then be used for effecting *N*-substitution which is preferably carried out with heating. It is advisable that the *N*-substitution take place in solution under an inert atmosphere such as nitrogen.

Alternatively, the compound of formula (II) may be reacted with carbazole in the presence of an alkali

metal hydroxide or alkali metal alkoxide in a polar solvent such as dimethylformamide, dimethylacetamide, water or the corresponding alkanol as appropriate; for example potassium *tert*-butoxide in *tert*-butanol.

The intermediates of formula (II) are readily obtained from the corresponding alcohol by conventional techniques. For example, the alcohol may be converted to the chloride by reaction with thionyl chloride. The alcohol may itself be made by reaction of a substituted alcohol of formula $Z—(CH_2)_3—OH$, wherein Z has the same meaning as in formula (II), with 3,5-*cis*-dimethylpiperazine.

The intermediates of formula (II) may also be obtained by an addition reaction involving a Michael-type condensation of a piperazine or carbazole, respectively with acrylic acid or a derivative thereof such as a acid halide, ester, amide or nitrile. The acid derivative can be hydrolysed to the acid which in turn can be reduced to the corresponding alcohol. The alcohol can then be converted to intermediates of formula (II) as previously described.

The "Carbazole", obtainable from the intermediates of the present invention, its pharmaceutically acceptable salts and solvates of these pharmaceutically acceptable salts are useful in the control of psychotic and aggressive conditions in humans. For example, it is useful for the prophylaxis and/or treatment of schizophrenia, mania or senile, involutional or organic psychoses as well as depressive psychosis.

The "Carbazole" may also be used in the prophylaxis and treatment of aggressive behaviour. Such behaviour may be associated with other disorders such as psychotic disorders and personality disorders as well as, in children, autism and hyperkinetics.

The "Carbazole", its pharmaceutically acceptable salts and solvates potentiates the locomotor activity of *d*-amphetamine, a property showed by most tricyclic antidepressant agents. This potentiation of *d*-amphetamine suggests that the "Carbazole" may have antidepressant activity in man.

A further use for the "Carbazole", its pharmaceutically acceptable salts and solvates of said salts is in the control of aggressive symptoms which may be associated with mentally retarded and/or behaviourally disturbed patients, or of aggression associated with epileptic disorders, acute or chronic organic brain syndromes, alcoholism, narcotic addiction and other forms of aggression of either known or unknown etiology.

The "Carbazole", the pharmaceutically acceptable salts and the pharmaceutically acceptable solvates are also indicated as having antidepressant activity at the same dosages as given heretofore.

It will be understood from the foregoing description that what we will claim in accordance with this invention is as follows:

A *cis*-dimethylpiperazine of formula (II)

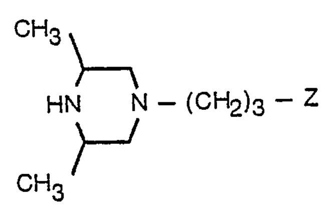

(II)

wherein Z is selected from halo, arylsulphonyloxy and alkylsulphonyloxy, or a salt thereof.

The compound of formula (II) according to claim 1, wherein Z is selected from chloro, *p*-toluenesulphonyloxy and methanesulphonyloxy, or a salt thereof.

3-(3,5-*cis*-Dimethylpiperazino)propyl chloride or a salt thereof.

The following Examples are given by way of illustration of the invention. In these Examples all temperature are in degrees Celsius and vacuum distillation was effected on a steam bath using a pressure of about 20 mm Hg, unless otherwise specified.

Example 1

Preparation of 9-[3-(3,5-*cis*-dimethylpiperazino)propyl]carbazole dihydrochloride hemihydrate.

A. 3-(3,5-*cis*-Dimethylpiperazino)propanol dihydrochloride

2,6-*cis*-Dimethylpiperazine (114.2 g, 1.0 mole), 3-chloropropanol (94.5 g, 1.0 mole), sodium carbonate (105 g) and ethylene glycol monomethyl ether (400 ml) were stirred under reflux for four hours. The reaction mixture was filtered warm, and the filtrate stripped of solvent by vacuum distillation.

The resulting solid was dissolved in ethanol (500 ml), and this solution saturated with dry hydrogen chloride. The white precipitate was collected, dried *in vacuo*, and used as the hydrochloride salt in the next step without further purification, m.p. 240—241°.

B. 3-(3,5-*cis*-Dimethylpiperazino)propyl chloride dihydrochloride

Thionyl chloride (300 g) was slowly added to 3-(3,5-*cis*-dimethylpiperazino)propanol dihydrochloride (200 g). The reaction was gently refluxed for an hour followed by removal of excess thionyl chloride by vacuum distillation. The resulting solid was washed with several portions of benzene then several portions of ether, m.p. 291—292°.

C. 9-[3-(3,5-*cis*-Dimethylpiperazino)propyl]carbazole dihydrochloride hemihydrate

To a slurry of sodium hydride (1.6 mole) made from 70 g of 57% dispersion in mineral oil and 200 ml of dimethylformamide (DMF) was added a solution of carbazole (83.5 g, 0.5 mole) in DMF (150 ml). When hydrogen was no longer evolved a slurry of 3-(3,5-*cis*-dimethylpiperazino)propyl chloride dihydrochloride (131 g, 0.5 mole) in DMF (200 ml) was added to the reaction at a slow rate. The reaction was run under nitrogen using a demand system with an oil bubbler. There was some foaming as the slurry was added and the rate of addition was adjusted to keep this to a minimum. After addition was completed the reaction was heated (90°—120°) for 16 to 18 hours, cooled, filtered and stripped of solvent by vacuum distillation. The viscous residue was warmed with 2 liters of 1*N* hydrochloric acid, cooled, and washed with ether (500 ml). The ether layer which contained any unreacted carbazole was discarded. The aqueous layer was treated with activated charcoal and filtered through Celite®. The filtrate was made basic to Alkacid® paper using sodium hydroxide, and then extracted with ether four times. Both ether and aqueous layers were clear at the end of the extraction. The ether layer way dried with anhydrous magnesium sulphate or molecular sieves and the hydrochloride salt of 9-[3-(3,5-*cis*-dimethylpiperazino)propyl]carbazole was precipitated by passing dry hydrogen chloride into the solution. The precipitate was collected and recrystallized from ethyl acetate or ethanol/ether mixture giving 9-[3-(3,5-*cis*-dimethylpiperazino)propyl]carbazole dihydrochloride hemihydrate (50 g), m.p. 288—290°C.

## Example 2

9-[3-(3,5-*cis*-Dimethylpiperazino)propyl]carbazole dihydrochloride hemihydrate

(A) 9-(3-Chloropropyl)carbazole

Carbazole (134 g, 0.8 mole) in dimethylformamide (1.5 litres) was slowly added to a slurry of sodium hydride (0.8 mole) made from 39 g of 50% dispersion in mineral oil and 500 ml of dimethylformamide while maintaining the temperature below 60°C. When hydrogen was no longer evolved the reaction temperature was lowered to around 5° with an ice bath and 1,3-dichloropropane (500 g, 4.4 mole) was added at a rate that did not exceed 10 ml per min. After all the 1,3-dichloropropane had been added the reaction mixture was slowly stirred while allowing it to come to room temperature (about 1.5 hours).

The reaction mixture was filtered to remove the sodium chloride that had formed, then stripped of solvent by vacuum (water pump) distillation. The thick syrup-like residue was then subjected to high vacuum distillation. The first fraction removed at 70°C and 300 microns was the excess 1,3-dichloropropane which was recovered for reuse. The second fraction distillation at 120°C and 100 microns was unreacted carbazole and some product, 38 g. The third fraction (b.p. 145° at 60 microns) was 9-(3-chloropropyl)carbazole, 107 g, 55% yield, m.p. 34—35°C.

| | |
|---|---|
| Calculated for $C_{15}H_{14}NCl$: | C 73.91, H 5.79, N 5.75 |
| Found: | C 73.85, H 5.81, N 5.72 |

(B) 9-[3-(3,5-*cis*-Dimethylpiperazino)propyl]carbazole dihydrochloride hemihydrate

9-(3-Chloropropyl)carbazole (12.2 g, 0.05 mole), 2,6-*cis*-dimethylpiperazine (5.7 g, 0.05 mole), potassium carbonate (10 g) and dimethylformamide (50 ml) were heated for an hour, cooled and filtered. The filtrate was stripped of solvent by vacuum (water pump) distillation and the residue was treated with 250 ml of 1N hydrochloric acid and the resulting solution was shaken with ether. The ether phase was discarded and the hydrochloric acid phase was treated with Celite®, filtered and vacuum distilled to dryness. The residue was recrystallized from an ethanol/ether mixture to give 9-[3-(3,5-*cis*-dimethylpiperazino)propyl]carbazole dihydrochloride hemihydrate (18 g, 90% yield) m.p. 288—290°C.

## Example 3

9-[3-(3,5-*cis*-Dimethylpiperazino)propyl]carbazole monohydrochloride

A slurry in toluene of 3-(3,5-*cis*-dimethylpiperazino)propyl chloride dihydrochloride (Example 1, step B) was quenched in a mixture of ice and 10*N* sodium hydroxide and the mixture adjusted to pH 13.5. The toluene phase was separated and four additional toluene extractions performed. After drying, the toluene solution solvent was removed to yield crude 3-(3,5-*cis*-dimethylpiperazino)propyl chloride as an oil.

Equimolar amounts of sodium methoxide and carbazole were reacted together in dimethylformamide followed by vacuum stripping to ensure removal of liberated methanol. This mixture was combined with an appropriate amount of the crude oil from the previous step and the resulting mixture heated at 100° for 15 hrs. Dilution with water of the cooled mixture thus obtained effected crystallisation of crude 9-[3-(3,5-*cis*-Dimethylpiperazino)propyl]carbazole. The latter was filtered, warmed with 1*N* hydrochloric acid and the resulting solution, after cooling, extracted with ether. The ether layer was discarded and the remaining aqueous layer neutralized to pH 7 with 5N sodium hydroxide. The resulting precipitate was filtered, washed with water and dried under reduced pressure to give 9-[3-(3,5-*cis*-dimethylpiperazino)propyl]carbazole monohydrochloride, m.p. 309—311° (dec.).

**Claims**

1. A *cis*-dimethylpiperazine of formula (II)

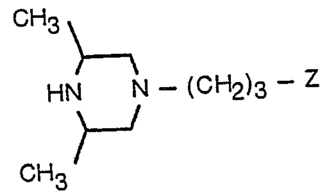

(II)

wherein Z is selected from halo, arylsulphonyloxy and alkylsulphonyloxy, or a salt thereof.

2. The compound of formula (II) according to claim 1, wherein Z is selected from chloro, *p*-toluenesulphonyloxy and methanesulphonyloxy, or a salt thereof.

3. 3-(3,5-*cis*-Dimethylpiperazino)propyl chloride or a salt thereof.

**Patentansprüche**

1. Cis-dimethylpiperazine der Formel

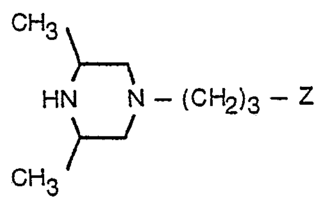

(II)

in der Z eine Halogen-, Arylsulfonyloxy- oder Alkylsulfonyloxy-Gruppe bedeutet oder eines seiner Salze.

2. Verbindung der Formel (II) gemäß Anspruch 1, in der p-Toluolsulfonyloxy oder Methansulfonyloxy bedeutet oder eines seiner Salze.

3. 3-(3,5-*Cis*-dimethylpiperazin)propylchlorid oder ein Salz davon.

**Revendications**

1. Une cis-diméthylpipérazine de formule (II)

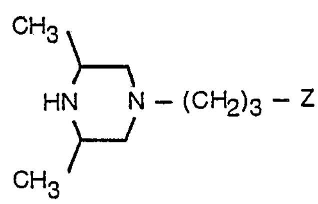

(II)

(dans laquelle Z est un atome d'halogène ou un groupe arylsulfonyloxy ou alkylsulfonyloxy), ou un sel d'une telle pipérazine.

2. Composé de formule (II) selon la revendication 1 dans lequel Z est un atome de chlore ou le groupe p-toluène-sulfonyloxy ou méthane-sulfonyloxy, ou un sel de ce composé.

3. Le chlorure de 3-(3,5-cis-diméthylpipérazino)propyle ou un sel de ce composé.